# EUROPEAN PATENT APPLICATION

(11) **EP 1 845 155 A1**
(43) Date of publication of application: **17.10.2007**
(21) Application number: 05805283.8
(22) Date of filing: 25.10.2005
(51) Int. Cl.: C12N 15/00, C12Q 1/37, C12M 1/34, G01N 21/78

(54) **PARTICLE FOR ENZYMATIC ACTIVITY DETECTION AND, UTILIZING THE SAME, METHOD OF ENZYMATIC ACTIVITY DETECTION AND ENZYMATIC ACTIVITY DETECTION TOOL**

(30) Priority: 13.01.2005 JP 2005006864
(71) Applicant: Kyushu Institute of Technology, Tobata-ku Kitakyushu-shi, Fukuoka 8048550 (JP)
(72) Inventor: NISHINO, Norikazu, Kitakyushu-shi Fukuoka 8080104 (JP); KATO, Tamaki, Kitakyushu-shi Fukuoka 8070843 (JP)
(74) Representative: HOFFMANN EITLE
(86) International application number: PCT/JP2005/019625
(87) International publication number: WO 2006/075429

(57) **Abstract**

An object is to provide a particle for detecting an enzyme activity wherein by measuring an intensity of a fluorescence wavelength in a sample solution, it is possible to not only detect the presence or absence of an enzyme but also accurately perform a quantitative analysis of the enzyme in the sample solution, handleability is excellent as well as measurement accuracy and quantitative property can be enhanced because the present invention is hardly influenced by absorbed moisture and weighing errors hardly occur, and further productivity is remarkably excellent because complicated steps such as a step of cleaving and purifying a synthesized peptide and a step of lyophilizing are not required upon production thereof.

The particle 1 for detecting the enzyme activity of the present invention comprises a particle 2 and a first compound 3 binding the particle 2 to one end, binding a first fluorescent group 4 to the other end and having a cleavage site by an enzyme 5 in a sample solution.

A particle formed of a synthetic resin or an inorganic material and having a constant surface area per unit weight

## Description

### Technical Field

The present invention relates to a particle for detecting an enzyme activity, which detects the enzyme activity, a method for detecting the enzyme activity and an enzyme activity detection device by the use thereof.

### Background of the Invention

In recent years, with advances in the field of medicine such as pathological diagnosis and in the field of research such as proteome analysis, there is a necessity to detect activities of multiple enzymes, and various techniques to measure enzyme activity in a solution have been studied using absorbance and fluorescence.
For example, in Patent document 1, "a fluorescent probe obtained by modifying both ends of a substrate peptide, which is specifically cleaved by caspase which is one type of protease, with fluorescent groups which cause fluorescence resonance energy transfer" is described.
Patent document 1: Japanese Published Unexamined Patent Application No. 2000-316598

### Summary of the Invention

### Problems to be Solved by the Invention

However, the above conventional art has had the following problems.
(1) In the technology disclosed in Patent document 1, the substrate peptide of the fluorescent probe in the solution is cleaved with the enzyme, and consequently, the fluorescence resonance energy transfer in the fluorescent group of the substrate peptide is released to change a fluorescence wavelength and a fluorescence intensity. Thus, by measuring this, the enzyme activity is detected. The fluorescent probe is supplied in a state of lyophilized powder or a solution. When the fluorescent probe supplied in the state of the powder is used, the fluorescent probe is prepared into the solution by weighing the powder in a given amount and dissolving in a solvent during use. However, if the solution is prepared incorrectly, the quantitative property in the detection of the enzyme activity is insufficient. Thus, the powder must be weighed at a high degree of accuracy as well as being able to confirm whether the fluorescent probe was completely dissolved in the prepared solution. The preparation before the measurement is complicated, and handleability is insufficient.
(2) With the recent advances in the field of medicine and in the field of research, types of enzymes required to be measured continue to increase, and along with this, the types and combinations of the required fluorescent probes exponentially increase. Thus, due to the above problems, both time and effort required for preparing the fluorescent probe solution before measurement have increased, the ratio of preparing work before the measurement occupied in the work for detecting enzyme activity has increased, and the burdens placed on measurers have increased.
(3) The lyophilized powder often has a hygroscopic property. In this case, due to moisture being absorbed into the powder, the powder is aggregated or gelatinized. Thus, the weighing accuracy is reduced, the concentration of the solution becomes incorrect and the quantitative property is insufficient.
(4) When the fluorescent probe in the solution is used, differently from the case of being supplied as the powder, there is no such problem as described above and the quantitative property is excellent. However, fluorescent substances are generally unstable in the solution. The fluorescent substance in the solution cannot be stored for a long time, and thus, storage stability is insufficient.
(5) In order to enhance the storage stability, the fluorescent probeisoftenstoredfrozen. However, it must be used by thawing upon measurement. Thus, the preparation of the fluorescent probe before measurement is troublesome and the work is complicated.
(6) When the fluorescent probe is produced, it is necessary that a given peptide is synthesized on the surface of a resin for peptide synthesis and subsequently the peptide is cleaved from the resin for peptide synthesis and then purified. Thus, the process is complicated and productivity is insufficient. When the fluorescent probe in the powder is produced, it is necessary that the purified peptide is lyophilized as well as sealed so as not to absorb moisture. The process is also complicated.

The present invention solves the above conventional problems, and aims to provide a particle for detecting enzyme activity, by which not only the presence or absence of the enzyme is detected but also the enzyme in a sample solution can be quantitatively analyzed with high accuracy, which is excellent in handleability as well as enhancing the measurement accuracy and quantitative property as absorbed moisture hardly influences and weighing errors hardly occur, and further productivity is excellent because complicated steps such as a step of cleaving and purifying a synthesized peptide or a lyophilizing step are not required when being produced.
The present invention also aims to provide a method for detecting an enzyme activity, for which the enzyme activity can be detected only by measuring the fluorescence intensity in the sample solution after contacting the sample solution containing the enzyme with the particle, which is excellent in measurement efficiency because measurement time can be shortened to enhance workability, the measurement efficiency can be enhanced exponentially because the enzyme activities in the sample solution containing multiple enzymes can be measured and analyzed completely in a short time by performing image analysis across a broad range using an image sensor, etc. utilizing a microplate such as a 96-well plate, etc., and searching and screening for useful substances leading to pharmaceuticals can be performed with high efficiency.
The present invention also aims to provide an enzyme activity detection device which can simply detect whether the enzyme having an activity is contained in the sample solution by measuring the fluorescence intensity in the sample solution which has passed through the device, and is excellent in handleability.

### Means for Solving the Problems

In order to solve the above conventional problems, the particle for detecting an enzyme activity, the method for detecting an enzyme activity and the enzyme activity detection device by the use thereof of the present invention have the following constitutions.
The particle for detecting the enzyme activity according to claim 1 of the present invention has the constitution comprising a particle and a first compound binding the particle to one end, binding a first fluorescent group to the other end and having a cleavage site by an enzyme.
By this constitution, the following actions are obtained.
(1) Because of comprising the particle and the first compound binding the particle to one end, binding the first fluorescent group to the other end and having the cleavage site by the enzyme, by contacting the particle with the sample solution containing the enzyme, the first fluorescent group is cleaved at the cleavage site by the enzyme to be liberated in the sample solution. Thus, the enzyme activity can be detected using the change in the fluorescence intensity in the sample solution as an indicator.
(2) If the first fluorescent group is not liberated and remains bound to the particle in the sample solution, when the fluorescence wavelength is measured, for example, irregular reflection occurs on the surface of the particle and the particle easily influences the measurement to reduce the quantitative property and the measurement accuracy. However, the first fluorescent group is liberated in the sample solution. Thus, when the fluorescence wavelength is measured, the particle hardly influences the measurement to enhance the quantitative property and the measurement accuracy.
(3) If a surface area per one particle is equal, an amount of the first compound, i.e., the amount of the first fluorescent group bound to one particle is an equivalent amount. Thus, the intensity of the fluorescence wavelength in the sample solution contacted with the particle in the same amount is proportional to the concentration of the enzyme in the sample solution. Therefore, by measuring the intensity of the fluorescence wavelength in the sample solution, not only is the presence or absence of the enzyme detected but also the enzyme in the sample solution can be quantitatively analyzed with good accuracy.
(4) Even if the first compound bound to the surface of the particle absorbs moisture, the size of the first compound is much smaller than the size of the particle, and the surface of the particle has fine asperities. Thus, the particles do not adhere to one another due to absorbed moisture. Therefore, the weighing accuracy can be enhanced without being aggregated or gelatinized, and the handleability is excellent.
(5) If the surface area of the particle is constant, the amount of the first compound bound to the surface of one particle is almost constant, and the weight of the first compound bound to the surface of the particle is much smaller than the weight of the particle. Thus, weighing errors hardly occur. If the weight of the particles is uniform, when a certain amount of the particles is weighed during use, the variation among the sample solutions due to the amount of the first compound, i.e., the first fluorescent group can be minimized to enhance the weighing accuracy, thereby being capable of enhancing the quantitative property of the fluorescence intensity and the accuracy of the quantitative analysis.
(6) The particle of the present invention can be produced by binding the first compound, the first fluorescent group to the surface of the particle using an automatic peptide synthesizer. Thus, it is not necessary that the synthesized peptide is cleaved from the resin for peptide synthesis and purified as in the case of producing the fluorescent probe using the resin for peptide synthesis. Thus, the production process is not complicated and the productivity is remarkably excellent. The particle binding the first compound in which the first fluorescent group was introduced can be commercialized by washing with a solvent and subsequently drying under reduced pressure without lyophilizing. Thus, the productivity can be enhanced.
(7) As having the first compound, by optimizing a distance from the particle to the cleavage site of the first compound, it is possible to allow the enzyme to act without being influenced by the particle. Thus, the enzyme activity can be detected accurately and detection accuracy can be enhanced.

Here, as the particle, those made from a synthetic resin such as polystyrene or an inorganic material such as silica gel or glass and formed into a roughly spherical, polyhedral or crushed shape are used. Among them, those formed into the roughly spherical shape or the polyhedral shape are suitably used. Because, since the shape of the particles can be standardized, the surface area of the particles per unit weight can be constant, the weight of the particle can be positively proportional to the surface area; if the amount of the fluorescent group bound to one particle is found, the amount of the fluorescent group bound to all particles can be estimated by making the weight of the particles uniform and weighing the particles; and the enzyme in the sample solution can be quantitatively analyzed from the measured fluorescence intensity.
As the material of the synthetic resin, those insoluble in a solvent such as halogenated hydrocarbons including chloroform and dichloromethane used for a condensation reaction for forming a peptide bond, esters such as ethyl acetate, polar organic solvents such as N,N-dimethylformamide and dimethylsulfoxide, ethers such as dioxane and tetrahydrofuran, alcohols such as methanol and ethanol, and pyridine are used. Commercially available carriers for solid phase organic synthesis such as the lantern series (registered trade name) manufactured by Mimotopes Pty. Ltd. can also be used.

A particle having a particle diameter of 0.05 to 0.5 mm is suitably used. This is because a peptide can be bound to the surface of a particle using a commercially available automatic peptide synthesizer.
The particle whose surface has been coated with polyethylene glycol is suitably used. This is because, the hydrophilicity and swelling property for the solvent can be enhanced.

As a first compound, a substrate peptide binding an amino acid or two or more amino acids via a peptide bond and comprising a special cleavage site by an enzyme is used. A substrate peptide having a molecular chain binding a peptide or another compound or molecule can also be used. The first compound can be synthesized using an ordinary peptide synthesis method such as a solid phase method in which the amino acid at the C terminus is immobilized on the particle and the peptide is extended from the C terminus. Also, a sequential extending method of sequentially extending from the C terminal side to the N terminal side of an objective amino acid sequence, or a fragment condensation method of extending by synthesizing multiple short peptide fragments and coupling between the peptide fragments can be used. Also, using a peptide synthesizer, it is possible to synthesize by introducing 9-fluorenylmethyloxycarbonyl (Fmoc) amino acid or t-butyloxycarbonyl (Boc) amino acid. Furthermore, it is also possible to generate a peptide bond using protease or synthesize utilizing gene engineering.

As the condensation method for forming the peptide bond in the first compound, publicly known methods can be used. For example, an azide method, an acid chloride method, an acid anhydride method, a mixed acid anhydride method, a DCC method, a DCC-additive method, an active ester method, a carbonyldiimidazole method, an oxidation-reduction method and a method using Woodward's reagent K are used.
Before performing the condensation reaction, by publicly known procedures, carboxyl groups and amino groups not involved in the reaction in the amino acid or the peptide can also be protected, or the carboxyl groups and the amino groups involved in the reaction can also be activated.

As the first fluorescent group, those in which the nature of the fluorescent group is changed to generate a change in the fluorescence wavelength or the fluorescence intensity before and after the first compound is cleaved at the cleavage site of the peptide bond by the enzyme are used. The fluorescent group in which the fluorescence wavelength or the fluorescence intensity is changed due to a concentration quenching phenomenon is also used. The concentration quenching phenomenon refers to the phenomenon that the fluorescence wavelength or the fluorescence intensity is changed when the fluorescent groups are close to one another on the particle and when the fluorescent groups are liberated in the sample solution to extend a distance between the fluorescent groups.
As such a fluorescent group, for example, 4-methylcoumaryl-7-amide (MCA), 7-amino-4-carboxymethylcoumarin (ACC), p-nitroanilide, α-naphthylamide, α-naphthyl ester and fluorescein or derivatives thereof are used.
By this, the fluorescence wavelength and the fluorescence intensity of the first fluorescent group liberated from the particle are different from those before the liberation. Thus, the amount cleaved by the enzyme can be measured using the fluorescence intensity in the particular wavelength region as the indicator.

The detectable enzymes can include all enzymes such as thrombin, plasmin, urokinase, elastase and collagenase which cleave the peptide.

The particle for detecting the enzyme activity according to claim 2 of the present invention has the constitution in which the first fluorescent group is fluorescein or the derivative thereof.
By this constitution, the following actions in addition to the actions obtained in claim 1 are obtained.
(1) In urine from subjects taking a drug or a vitamin preparation, miscellaneous fluorescent substances derived from the drug and the vitamin are excreted. These fluorescent substances often have a fluorescence wavelength around 400 nm. Thus, when urine is directly used as a sample solution, the first fluorescent group having the fluorescence wavelength around 400 nm cannot be used. This is because the fluorescence derived from the fluorescent substance contained in the drug cannot be distinguished from the fluorescence from the first fluorescent group liberated in the sample solution by enzyme activity. Fluorescein that emits fluorescence around 525 nm, can be distinguished from the fluorescence wavelength of the fluorescent substance contained in the drug, and is excellent in versatility because urine from subjects taking the drug or vitamin preparation can be directly used as the sample solution.

Here, as the fluorescein derivative, fluorescein isothiocyanate (FITC) is used.

The particle for detecting the enzyme activity according to claim 3 of the present invention has a constitution comprising a second compound binding a second fluorescent group to one end, binding a quenching agent to an atomic group at the other end and having a cleavage site by an enzyme, and a particle bound to the atomic group or the quenching agent at the other end of the second compound.
By this constitution, the following actions in addition to the actions obtained in claim 1 are obtained.
(1) When the peptide bond is cleaved at the cleavage site of the second compound by the enzyme, the distance between the quenching agent and the second fluorescent group extends to change the fluorescence spectrum of the second fluorescent group. Thus, this spectral change can be used as an indicator for measuring the enzyme activity. This enables detection of an enzyme activity using the change in the fluorescence intensity as the indicator.

Here, as the quenching agent, the substance which absorbs the light corresponding to a fluorescence excitation wavelength of the second fluorescent group, the substance which forms a non-luminescent complex by loosely binding with the second fluorescent group, and the substance which causes the fluorescence resonance energy transfer in the second fluorescent group are used.
The fluorescence resonance energy transfer refers to the phenomenon that when the second fluorescent group and the quenching agent are present inclose locations, if the excitation spectrum of the quenching agent (acceptor) and the fluorescent spectrumof the second fluorescent group (donor) are overlapped, the fluorescence of the second fluorescent group which should be observed in nature is attenuated because the quenching agent deprives the excitation energy when the energy of the excitation wavelength of the second fluorescent group is given.

As the second fluorescent group and the quenching agent, the combination of the donor and the acceptor where the fluorescence resonance energy transfer occurs can be used. For example, the quenching agent which is the atomic group having an absorption zone in the wavelength region overlapped with the fluorescence wavelength of the second fluorescent group is used. Specifically, the combination of dinitrophenyl (Dnp) with (7-methoxycoumarin-4-yl)acetyl (MOAc), anthraniloylbenzyl (ABz) or N-methyl anthranilic acid (Nma), the combination of Dabsyl with EDANS (5-(2'-aminoethyl)aminonaphthalene-1-sulfonic acid), the combination of tryptophan (Trp) with 5-dimethylamino-1-naphthalene sulfonic acid (Dns), the combination of carboxydichlorofluorescein (CDCF) with carboxymethylrhodamine (CTMR), the combination of carboxydichlorofluorescein (CDCF) with carboxy X-rhodamine (CXR), and the combination of Lucifer yellow with carboxymethylrhodamine (CTMR) are used.

As the second compound, the substrate peptide binding the amino acid or two or more amino acids via the peptide bond and comprising the particular cleavage site by the enzyme is used. The substrate peptide having the molecular chain binding the peptide or another compound or molecule can also be used.
The atomic group or the quenching agent in the second compound may be directly bound to the particle, or may be bound to the particle via the molecular chain binding the peptide or another compound or the molecule.
In the bond between the quenching agent and the particle, the bond between the atomic group and the quenching agent in the second compound and the bond between the atomic group in the second compound and the particle, an amide bond, an ester bond, an ether bond, a thioether bond and an urethane bond which are not cleaved by the enzyme are used. This is because, the fluorescent spectrum of the second fluorescent group is changed even by the quenching agent being cleaved by an enzyme to be liberated from the particle, but the enzyme activity depending on the amino acid sequence of the second compound cannot be detected.

It is desirable that the length of binding between the second fluorescent group and the quenching agent bound to the second compound is 100 angstroms or less. Because as the distance of binding between the second fluorescent group and the quenching agent lengthens, the change in the fluorescence intensity tends to become small, and when the distance exceeds 100 angstroms, this tendency becomes remarkable and the change in the fluorescence intensity becomes remarkably small and the sensitivity is reduced.

Enzymes whose activity can be detected can include endopeptidase which cleaves the internal peptide bond, e.g., serine proteases such as trypsin, chymotrypsin, thrombin, plasmin, kallikrein, urokinase and elastase, aspartic acid proteases such as pepsin, cathepsin D, rennin and chymosin, metalloproteases such as carboxypeptidase A, B, collagenase and thermolysin, cysteine proteases such as cathepsin B, H, L and calpain; blood coagulation system proteases; complement system proteases; and hormone processing enzymes.

The particle is the same as that described in claim 1. Thus, its description is omitted.

The invention according to claim 4 of the present invention is the particle for detecting the enzyme activity according to any one of claims 1 to 3, and has the constitution where the first compound or the second compound has an acetylated amino acid residue.
By this constitution, the following actions in addition to the actions obtained in any one of claims 1 to 3 are obtained.
(1) The first compound or the second compound has the acetylated amino acid residue. Thus, when the substrate peptide is acetylated, even if the particular enzyme which does not cleave the substrate peptide or has a significantly lowered cleavage activity is contacted, no change in the fluorescence intensity is detected. However, when the sample solution containing this particular enzyme and a deacetylase which liberates an acetyl group from the acetylated peptide is contacted with the particle for detecting the enzyme activity, the acetyl group is liberated from the peptide by the deacetylase and the peptide whose acetyl group has been liberated is cleaved with the particular enzyme and emits the fluorescence having the particular wavelength. Thus, using the fluorescence intensity in the sample solution as the basis, it is possible to detect to what extent an inhibitory substance for the deacetylase activity, which is a candidate compound of an anticancer agent is present in the sample solution. Thus, the candidate substance of the anticancer agent can be screened. A histone deacetylase is involved in progress of cell cycle and differentiation, and has important actions to regulate the expression of various genes by changing the nucleosome structure. It has been reported that collapse of this regulation is associated with certain cancers, and it has been known that inhibitors of the histone deacetylase are effective in the treatment of tumors and leukemia.

The method of acetylating the amino acid residue in the substrate peptide in the first compound or the second compound includes the method of acetylating the amino acid blocking α-amino group and side chain amino group with protection groups using acetic acid anhydride or N-hydroxysuccinimide acetate.

The method for detecting the enzyme activity according to claim 5 of the present invention has the constitution comprising (a) a contact reaction step of contacting the sample solution containing the enzyme with the particle for detecting the enzyme activity according to any one of claims 1 to 3 and (b) a fluorescence measurement step of measuring the fluorescence in the sample solution reacted with the particle for detecting the enzyme activity.
By this constitution, the following actions are obtained.
(1) The enzyme activity can be detected only by measuring the fluorescence intensity in the sample solution after contacting and reacting the sample solution containing the enzyme with the particle for detecting the enzyme activity. Thus, the measurement time can be shortened, the workability can be enhanced and the measurement efficiency can be enhanced.
(2) Since the enzyme activity is detected by measuring the fluorescence, the detection sensitivity and the measurement accuracy can be enhanced.
(3) The enzyme activities in the sample solution containing the multiple enzymes can be completely measured and analyzed in a short time by placing the particles for detecting the enzyme activitybinding the fluorescent groups to respective different peptides in wells in a 96-well microplate for fluorescence measurement, injecting the sample solution in each well and performing image analysis across a broad range using the image sensor. Thus, the measurement efficiency can be exponentially enhanced.

Here, as the sample solution containing the enzyme, it is possible to use body fluids such as blood and urine subjected to pathological diagnosis and culture media. As the sample solution, a body sample (blood, etc.) can be directly used, or can be used by removing blood cell components by a filter or centrifugation. The sample solution with condition setting (pH adjustment, activator introduction, etc.) so that the enzyme expresses its activity based on the body sample can also be used. As a pH adjuster, it is possible to add a buffer such as Tris-HCl and Hepes-KOH as a reaction buffer. It is also possible to add salts and activation protecting agents required for the expression of the enzyme activity.

In the fluorescence measurement in the sample solution, it is possible to use the method in which the light from a light emitting element such as a light emitting diode is passed through the filter passing the excitation wavelength of the fluorescent group and irradiated to the sample solution and the fluorescence intensity is measured by a light receiving element such as a CCD disposed in aposition capable of detecting the fluorescence in the sample solution, in addition to the use of a fluorescence spectrophotometer.

The method for detecting the enzyme activity according to claim 6 of the present invention has the constitution comprising (a) a contact reaction step of contacting and reacting the particle for detecting the enzyme activity according to claim 4 with the sample solution containing the deacetylase which liberates the acetyl group from the first compound or the second compound having the acetylated amino acid residue and a particular enzyme which selectively cleaves the peptide bond of the amino acid residue whose acetyl group has been liberated, and (b) a fluorescence measurement step of measuring the fluorescence in the sample solution reacted with the particle for detecting the enzyme activity.
By this constitution, the following actions in addition to the actions according to claim 5 are obtained.
(1) When the first compound or the second compound has been acetylated, if the sample solution containing the particular enzyme which does not cleave the substrate peptide or has a significantly lowered cleavage activity and the deacetylase which liberates the acetyl group from the acetylated peptide is contacted with the particle for detecting the enzyme activity, the acetyl group is liberated from the peptide by deacetylase and the peptide whose acetyl group has been liberated is cleaved with the particular enzyme and exhibits the fluorescence having the particular wavelength. Thus, using the fluorescence intensity in the sample solution as the basis, it is possible to detect to what extent the inhibitory substance for the histone deacetylase activity, which is the candidate compound of the anticancer agent is present in the sample solution. Thus, the candidate substance of the anticancer agent can be screened easily and promptly. As an existing method for measuring the histone deacetylase activity, an extremely complicated method, e.g., the method of using histone metabolically radiolabeled by adding radiolabeled acetic acid into the medium of cultured cells or the method using acetylated lysine labeled with a fluorescent substance and separating a reaction product by reverse phase HPLC for measurement has been used.

Here, the deacetylase can include the histone deacetylase.

The particular enzyme can include lysyl endopeptidase, plasmin, calpain, trypsin, metalloprotease, Armillariamellea protease. These peptidases are suitably used because they do not cleave the peptide in which the amino group in lysine residue has been acetylated or the cleavage activity for it is significantly lowered.

The sample solution is the same as that described in claim 5, and thus, its description is omitted.
The concentration of the particular enzyme in the sample solution is set to the concentration at which the substrate peptide in the first compound or the second compound is sufficiently cleaved under a given condition. For example, even when all of the substrate peptide can be cleaved by deacetylation, it is desirable to make the concentration at which sufficient cleavage activity is present under the given measurement condition. This is because it is prevented that the cleavage activity of the particular enzyme is short and the inhibitory substance effect on the deacetylase activity is estimated low.

The enzyme activity detection device according to claim 7 of the present invention has the constitution comprising a vessel comprising a liquid inlet formed upstream of a liquid passing path and a liquid outlet formed downstream of the liquid inlet, and the particles for detecting the enzyme activity according to any one of claims 1 to 4, housed in the liquid passing path and retained in the vessel.
By this constitution, the following actions are obtained.
(1) Since the device comprises the vessel comprising the liquid inlet formed upstream of the liquid passing path and the liquid outlet formed downstream of the liquid inlet, and the particles for detecting the enzyme activity filled in the liquid passing path and retained in the vessel, the sample solution such as urine is placed from the liquid inlet into the vessel and contacted and reacted with the particles for detecting the enzyme activity, and subsequently the fluorescence intensity in the sample solution discharged from the liquid outlet is measured, thereby being capable of simply detecting whether the enzyme having the activity is contained in the sample solution using the fluorescence intensity in the sample solution as the indicator because when the enzyme having the activity is contained in the sample solution, the fluorescent group is liberated in the sample solution.
(2) After finishing the measurement, a new measurement can be performed by replacing the whole vessel with a new one, and thus the handleability is excellent.
(3) The fluorescence intensity is measured in the sample solution discharged from the liquid outlet after contacting with the particles for detecting the enzyme activity. Thus, the sample solution can be measured without being influenced by the fluorescence from the particles for detecting the enzyme activity. Therefore, the fluorescent group can be selected without considering the fluorescence change caused by being liberated from the particles for detecting the enzyme activity, and thus flexibility is excellent. Because, when the fluorescent group whose fluorescence change before and after the liberation from the particle is small is used, the case of measuring the fluorescence wavelength in the sample solution in a trace amount using the microplate for fluorescence measurement is easily influenced by the fluorescence wavelength of the particles, the quantitative property and the measurement accuracy are reduced, and thus, the fluorescent group must be selected in consideration of the fluorescence change.

### Effects of the Invention

According to the particles for detecting the enzyme activity and the method for detecting the enzyme activity and the enzyme activity detection device by the use thereof of the present invention as above, the following effects are obtained.
According to the invention of claim 1:
(1) Since the intensity of the fluorescence wavelength in the sample solution contacted with the particles in the same amount is proportional to the concentration of the enzyme in the sample solution, it is possible to provide the particle for detecting the enzyme activity which not only can detect the presence or absence of the enzyme but also can accurately perform the quantitative analysis of the enzyme in the sample solution by measuring the intensity of the fluorescence wavelength in the sample solution. It is also possible to provide the particle for detecting the enzyme activity which hardly influences measurement of the fluorescence wavelength to afford a high quantitative property and a high measurement accuracy.
(2) It is possible to provide the particle for detecting the enzyme activity which can enhance the weighing accuracy as well as being excellent in handleability because the particles are not adhered to one another by absorbed moisture and are not aggregated and gelatinized.
(3) It is possible to provide the particle for detecting the enzyme activity where weighing errors of the particles hardly occur, when the particles are weighed in a certain amount during use, the variation of the amounts of the first compound and the first fluorescent group among the sample solutions can be minimized to enhance the weighing accuracy and the quantitative property.
(4) It is possible to provide the particle for detecting the enzyme activity which is remarkably excellent in productivity because the particle can be produced by binding and synthesizing the first compound and the first fluorescent group on the particle surface using an automatic peptide synthesizer and thus, complicated steps such as the steps of cleaving, purifying, and lyophilizing the peptide are not required.
(5) It is possible to provide the particle for detecting the enzyme activity which has a high detection accuracy of the enzyme activity because the distance between the particle and the cleavage site is optimized to allow the enzyme to act without being influenced by the particle.

According to the invention of claim 2, in addition to the effects of claim 1:
(1) It is possible to provide the particle for detecting the enzyme activity which is excellent in versatility because the fluorescence wavelength of fluorescein can be distinguished from the fluorescence wavelength of the fluorescent substances contained in drugs, and thus, urine from subjects taking a drug or a vitamin preparation can be directly used as the sample solution.

According to the invention of claim 3, in addition to the effects of claim 1 or 2:
(1) It is possible to provide the particle for detecting the enzyme activity where when the peptide bond in the substrate peptide is cleaved by the enzyme, no interaction occurs because the distance between the second fluorescent group and the quenching agent is extended, and thus the spectral change to the fluorescent spectrum from the second fluorescent group can be used as the indicator for measuring the enzyme activity, thereby being capable of detecting the enzyme activity using the change in the fluorescence intensity as the indicator.

According to the invention of claim 4, in addition to the effects in any one of claims 1 to 3:
(1) It is possible to provide the particle for detecting the enzyme activity where when the sample solution containing the particular enzyme and the deacetylase which liberates the acetyl group from the acetylated peptide is contacted with the particles for detecting the enzyme activity, the acetyl group is liberated from the peptide by the deacetylase and the peptide whose acetyl group has been liberated is cleaved with the particular enzyme and exhibits the fluorescence having the particular wavelength, thus using the fluorescence intensity in the sample solution as the basis, it is possible to detect to what extent the inhibitory substance for the deacetylase activity is present in the sample solution, thus, the inhibitory substance for the deacetylase activity can be screened promptly, and useful substances leading to the discovery of drugs for anticancer agents and leukemia therapeutic agents can be searched and screened highly efficiently.

According to the invention of claim 5:
(1) It is possible to provide the method for detecting the enzyme activity which is excellent in measurement efficiency because the enzyme activity can be detected only by measuring the fluorescence intensity of the sample solution after contacting and reacting the sample solution containing the enzyme with the particles for detecting the enzyme activity, thereby being capable of shortening the measurement time and enhancing the workability.
(2) It is possible to provide the method for detecting the enzyme activity where the detection sensitivity and the measurement accuracy can be enhanced because the enzyme activity is detected by fluorescence measurement.
(3) It is possible to provide the method for detecting the enzyme activity where the enzyme activities in the sample solution containing the multiple enzymes can be completely measured and analyzed in a short time by placing the particles for detecting the enzyme activitybinding the fluorescent groups to respective different types of peptides in wells in the 96-well microplate for fluorescence measurement, injecting the sample solution in each well and performing image analysis across a broad range using the image sensor, thus the measurement efficiency can be exponentially enhanced, and useful substances leading to pharmaceuticals can be searched and screened highly efficiently.

According to the invention of claim 6, in addition to the effects of claim 5:
(1) It is possible to provide the method for detecting the enzyme activity where the acetyl group is liberated from the peptide by the deacetylase and the peptide whose acetyl group has been liberated is cleaved with the particular enzyme and exhibits the fluorescence having the particular wavelength, thus using the fluorescence intensity in the sample solution as the basis, it is possible to detect to what extent the inhibitory substance for the activity of the histone deacetylase which is the candidate compound of anticancer agents is present in the sample solution, thus the candidate compound such as anti-cancer agents can be screened easily and promptly, and useful substances leading to the discovery of drugs can be searched and screened highly efficiently.

According to the invention of claim 7:
(1) It is possible to provide the enzyme activity detection device for which it can be simply detected whether the enzyme having the activity is contained in the sample solution because the sample solution such as urine is placed from the liquid inlet into the vessel and contacted and reacted with the particles for detecting the enzyme activity, subsequently the fluorescence intensity in the sample solution discharged from the liquid outlet is measured, and at that time in the case of containing the enzyme having the activity in the sample solution, the fluorescent group is liberated into the sample solution to change the fluorescence intensity.
(2) It is possible to provide the enzyme activity detection device where after finishing the measurement, the new measurement can be performed by replacing the whole vessel with a new one, and thus the handleability is excellent.
(3) It is possible to provide the enzyme activity detection device which is excellent in flexibility because the sample solution can be measured without being influenced by the fluorescence from the particles for detecting the enzyme activity and the fluorescent group can be selected without considering the fluorescence change caused by the liberation from the particles for detecting the enzyme activity.

### Brief Description of the Drawings

FIG. 1 is a schematic view showing a principle for detecting an enzyme activity of a particle for detecting the enzyme activity in Embodiment 1;
FIG. 2 is a schematic view showing a principle for detecting an enzyme activity of a particle for detecting the enzyme activity in Embodiment 2;
FIG. 3 is a schematic view showing a modified example of the particle for detecting the enzyme activity in Embodiment 2;
FIG. 4 is a schematic view showing a principle for detecting an enzyme activity of a particle for detecting the enzyme activity in Embodiment 3;
FIG. 5 is a schematic view showing a principle for detecting an enzyme activity of a particle for detecting the enzyme activity in Embodiment 4; and
FIG. 6 is a schematic view showing an enzyme activity detection device in Embodiment 5.

### Description of Symbols

1: Particle for detecting enzyme activity
2: Particle
3: First compound
4: First fluorescent group
5: Enzyme
6: First fluorescent group
10: Particle for detecting enzyme activity
11: Second compound
12: Quenching agent
13: Second fluorescent group
14: Enzyme
15: Second fluorescent group
20: Particle for detecting enzyme activity
21: First compound
22: First fluorescent group
23: Deacetylase
24: Particular enzyme
30: Particle for detecting enzyme activity
31: Second compound
32: Quenching agent
33: Second fluorescent group
34: Deacetylase
35: Particular enzyme
36: Second fluorescent group
40: Enzyme activity detection device
41: Vessel
42: Liquid passing path
43: Liquid inlet
44: Liquid outlet
45, 46: Filter
47: Particle for detecting enzyme activity

### Detailed Description of the Invention

The best modes for carrying out the present invention will be described below with reference to the drawings.

### (Embodiment 1)

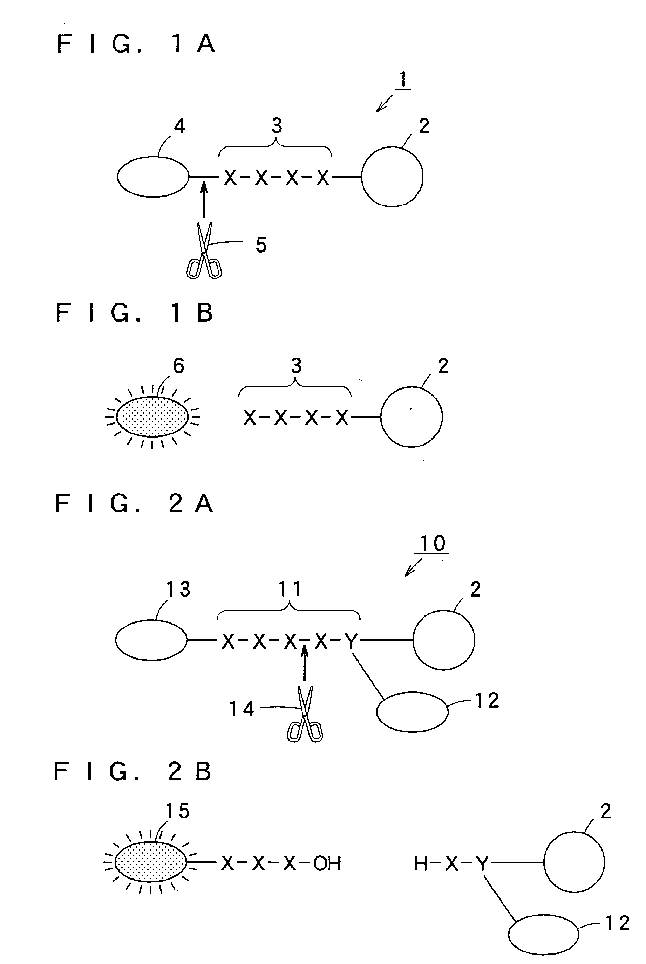

FIG. 1 is a schematic view showing a principle for detecting an enzyme activity of a particle for detecting the enzyme activity in Embodiment 1 of the present invention.
In the figure, the numeral 1 represents a particle for detecting an enzyme activity, 2 represents a particle obtained by forming a synthetic resin (polystyrene, etc.) or glass insoluble in a solvent such as halogenated hydrocarbons or esters into a roughly spherical or polyhedral shape, and 3 represents a first compound such as peptide or amino acid binding the particle 2 at one end. X in the first compound 3 represents any of amino acid residues. The numeral 4 represents a first fluorescent group such as 4-methylcoumaryl-7-amide (MCA) or fluorescein isothiocyanate (FITC) which is one of the fluorescent groups which is bound to the other end of the first compound 3 and generates the change in fluorescence wavelength or fluorescence intensity before and after a peptide bond with the first compound 3 or a cleavage site of the first compound 3 is cleaved with an enzyme 5 described later. The numeral 5 represents the enzyme which selectively cleaves the peptide bondbetween the first fluorescent group 4 and the first compound 3 or a substrate peptide in the first compound 3 at the particular cleavage site and is the enzyme such as metalloprotease having a substrate specificity in a sample solution. The numeral 6 represents the first fluorescent group which is liberated by being cleaved with the enzyme 5 at the cleavage site of the first compound 3 to change the fluorescence wavelength.

The particle 1 for detecting the enzyme activity constituted as above can be synthesized using an ordinary peptide synthesis method such as a solid phase method in which the first compound 3 such as a peptide is extended from the C terminus, the sequential extending method of sequentially extending from the C terminal side to the N terminal side of the objective amino acid sequence, the fragment condensation method of extending by synthesizing multiple short peptide fragments and coupling between the peptide fragments, and the method of synthesizing by introducing Fmoc or Boc using the peptide synthesizer.

For the particle for detecting the enzyme activity in Embodiment 1 constituted as above, the principle for detecting the enzyme activity will be described below.
The first fluorescent group 4 such as 4-methylcoumaryl-7-amide (MCA) or fluorescein isothiocyanate (FITC) in the particle 1 for detecting the enzyme activity shown in FIG. 1A is a non-fluorescent substance in a particular wavelength region, and when present on the surface of the particle 2 at high density, its fluorescence intensity is weak due to concentration quenching. When the sample solution containing the enzyme 5 is contacted and reacted with this particle 1 for detecting the enzyme activity, the enzyme 5 such as metalloprotease having the substrate specificity selectively cleaves the peptide bond between the first fluorescent group 4 and the first compound 3 or the particular cleavage site of the substrate peptide in the first compound 3.
The first fluorescent group 6 liberated from the first compound 3 becomes a fluorescent substance such as 7-amino-methylcoumarin (AMC), and the effect of the concentration quenching is reduced by liberating from the particle 2. The fluorescence wavelength or the fluorescence intensity in the particular wavelength region in the first fluorescent group 6 is different from those in the first fluorescent group 4 bound to the first compound 3 via the peptide bond. Thus, it is possible to detect the enzyme activity using the change in the fluorescence intensity in the sample solution as the indicator (see FIG. 1B).

The particle for detecting the enzyme activity is constituted as above, and thus, the following actions are obtained.
(1) Since the particle of the present invention comprises the particle 2 and the first compound 3 binding the particle 2 at one end and the first fluorescent group 4 at the other end and having the cleavage site by the enzyme 5 in the sample solution, the enzyme activity can be detected by contacting and reacting the particle 2 with the sample solution containing the enzyme 5 and using a change in the fluorescence intensity in the sample solution as the indicator.
(2) Even if the first compound 3 bound to the surface of the particle 2 absorbs moisture, the size of the first compound 3 is much smaller than the size of the particle 2, and the surface of the particle 2 has fine asperities. Thus, the particles 2 do not adhere to one another due to absorbed moisture. Therefore, the weighing accuracy can be enhanced without being aggregated or gelatinized, and the handleability is excellent.
(3) The particle of the present invention can be produced by binding and synthesizing the first compound 3 and the first fluorescent group 4 on the surface of the particle 2 using the automatic peptide synthesizer. Thus, the productivity can be enhanced because no step of cleaving and purifying the synthesized peptide is required.
(4) As having the first compound 3, the distance between the particle 2 and the cleavage site (peptide bond between the first fluorescent group 4 and the first compound 3 in this embodiment) is optimized to allow the enzyme 5 to act without being influenced by the particle 2. Thus, the enzyme activity can be accurately detected and the detection accuracy can be enhanced.

### (Embodiment 2)

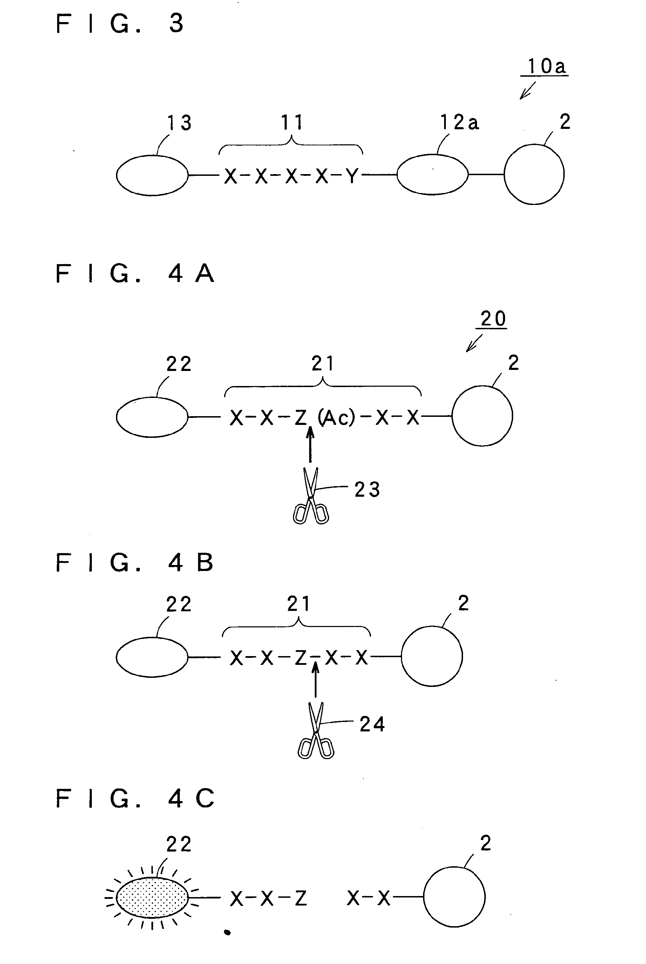

FIG. 2 is the schematic view showing the principle for detecting the enzyme activity of the particle for detecting the enzyme activity in Embodiment 2 of the present invention. The same symbols are given to the same items as in Embodiment 1, and description is omitted.
In the figure, the numeral 10 represents the particle for detecting the enzyme activity in Embodiment 2, 11 represents a second compound which is the substrate peptide having the cleavage site or the molecular chain binding the peptide or another compound or the molecule to the substrate peptide. X and Y in the second compound 11 represent atomic groups of any amino acid residues, and the atomic group Y at the end is bound to the particle 2. The numeral 12 represents the quenching agent such as dinitrophenyl (Dnp) or 5-dimethylamino-1-naphthalenesulfonic acid (Dns) boundto the atomic group Y in the second compound 11. The numeral 13 represents the second fluorescent group such as (7-methoxycoumarin-4-yl) acetyl (MOAc) or tryptophan (Trp) which causes the fluorescence resonance energy transfer with the quenching agent 12 introduced into other end in the second compound 11. The quenching agent 12 and the second fluorescent group 13 are bound at the distance (100 angstroms or less) in which they interact with each other to influence their fluorescence. The numeral 14 represents the enzyme such as serine protease, aspartic acid protease, metalloprotease and cysteine protease, and 15 represents the second fluorescent group which is liberated by being cleaved with substrate specificity of the enzyme 14 at the particular cleavage site of the second compound 11 to change the fluorescence wavelength.

For the particle for detecting the enzyme activity in Embodiment 2 constituted as above, the principle for detecting the enzyme activity will be described below.
Since the quenching agent 12 and the second fluorescent group 13 in the particle 10 for detecting the enzyme activity shown in FIG. 2A are bound at the distance in which they interact with each other to influence their fluorescence, the fluorescence spectrum of the quenching agent 12 and the fluorescence spectrum of the second fluorescent group 13 are overlapped. When the energy at the excitation wavelength of the second fluorescent group 13 is exposed, the fluorescence of the second fluorescent group 13 which should be inherently observed is attenuated.
When the sample solution containing the enzyme 14 is contacted and reacted with the particle 10 for detecting the enzyme activity, the enzyme 14 having the substrate specificity cleaves the peptide bond in the second compound 12.
When the second fluorescent group 13 is liberated from the particle 2, the interaction to influence the fluorescence between the second fluorescent group 13 and the quenching agent 12 is not observed. Thus, when the energy having the excitation wavelength of the second fluorescent group 13 is exposed to the sample solution, the fluorescence wavelength of the second fluorescent group 15 which has not been observed before contacting with the sample solution is observed, and is different from the fluorescence wavelength before the reaction with the enzyme 14. Thus, the enzyme activity can be detected using the change in the fluorescence intensity as the indicator (see FIG. 2B).

The particle for detecting the enzyme activity in Embodiment 2 is constituted as above, and thus, the following actions are obtained in addition to the actions described in Embodiment 1.
(1) It becomes possible to set the fluorescence wavelength of the second fluorescent group 13 to the visible region by selecting the quenching agent 12 and the second fluorescent group 13. Thus, it becomes possible to measure using a visible light detection apparatus such as a commercially available CCD camera, and the invention is excellent in versatility.
(2) The amino acid sequence can be freely selected for the second compound 11. Thus, as the enzyme 14, the enzyme which selectively cleaves the peptide bond at the N terminal side and the C terminal side, and the enzyme such as elastase having the low substrate specificity for an individual amino acid and requiring a relatively long peptide chain for expressing the cleavage action can also be detected. Thus, the types of the enzyme capable of being detected can be increased, and the invention is excellent in practical applicability.

FIG. 3 is the schematic view showing the modified example of the particle for detecting the enzyme activity in Embodiment 2. The same symbols are given to the same items as in Embodiment 2, and description is omitted.
In the figure, 10a represents the particle for detecting the enzyme activity in the modified example, 12a represents the quenching agent bound to the atomic group Y such as amino acid residues at the end of the second compound 11, and the quenching agent 12a is also bound to the particle 2.
Also in the case of the particle 10a for detecting the enzyme activity in the modified example, as described in Embodiment 2, when the peptide bond is cleaved at the cleavage site of the second compound 11 by the enzyme, the distance between the quenching agent 12a and the second fluorescent group 13 extends to change the fluorescence spectrum of the second fluorescent group 13. Thus, this spectral change can be used as the indicator for measuring the enzyme activity, and the enzyme activity can be detected using the change in the fluorescence intensity in the sample solution as the indicator.

### (Embodiment 3)

FIG. 4 is the schematic view showing the principle for detecting the enzyme activity of the particle for detecting the enzyme activity in Embodiment 3 of the present invention. The same symbols are given to the same items as in Embodiment 1, and description is omitted.
In the figure, the numeral 20 represents the particle for detecting the enzyme activity in Embodiment 3, and 21 represents the first compound such as a peptide bound to the surface of the particle 2 at one end. X in the first compound 21 represents any amino acid residue and Z represents the amino acid residue such as acetylated lysine. The numeral 22 represents the first fluorescent group such as fluorescein isothiocyanate (FITC) bound to the amino acid residue X in the first compound 21 via the peptide bond, 23 represents the deacetylase such as histone deacetylase in the sample solution, which catalyzes the reaction to liberate the acetyl group (Ac) from the acetylated amino acid residue Z, and 24 represents the particular enzyme such as metalloprotease in the sample solution, which selectively cleaves the peptide bond of the amino acid residue Z in the first compound 21.

Concerning the particle for detecting the enzyme activity in Embodiment 3 constituted as above, the principle for detecting the enzyme activity will be described below.
The first fluorescent group 22 such as fluorescein isothiocyanate (FITC) in the particle 20 for detecting the enzyme activity shown in FIG. 4A has weak fluorescence intensity due to the concentration quenching. When the sample solution containing the deacetylase 23 and the particular enzyme 24 is contacted and reacted with this particle 20 for detecting the enzyme activity, the deacetylase 23 liberates the acetyl group (Ac) from the amino acid residue Z in the first compound 21. The particular enzyme 24 such as metalloprotease does not cleave the peptide bond or has a significantly lowered cleavage activity and exhibits almost no activity when the amino acid residue Z in the first compound 21 is acetylated.
When the acetyl group is liberated from the amino acid residue Z in the first compound 21, the particular enzyme 24 such as metalloprotease selectively cleaves the C terminal side of the amino acid residue Z whose acetyl group (Ac) has been liberated as shown in FIG. 4B.
The fluorescence wavelength or the fluorescence intensity in the particular wavelength region of the first fluorescent group 22 liberated from the particle 2 is different from that of the first fluorescent group 22 bound to the particle 2. Thus, the enzyme activity can be detected using the change in the fluorescence intensity in the sample solution as the indicator (see FIG. 4C).
Therefore, when the sample solution contains the inhibitory substance for the deacetylase activity, which inhibits the activity of the deacetylase 23, by measuring and comparing each fluorescence intensity in the case where the sample solution does not contain the inhibitory substance for the deacetylase activity, it is possible to determine whether the inhibitory substance for the deacetylase activity is effective or not.

Since the particle for detecting the enzyme activity in Embodiment 3 is constituted as above, the following action is obtained.
(1) Even when contacted with the particular enzyme 24 which does not cleave the peptide bond or has a significantly lowered cleavage activity when the amino acid residue Z in the first compound 21 is acetylated, no change in the fluorescence intensity is detected. However, when the particle 20 for detecting the enzyme activity is contacted with the sample solution containing the particular enzyme 24 and the deacetylase 23 which liberates the acetyl group from the acetylated first compound 21, the acetyl group is liberated from the first compound 21 by the deacetylase 23, the first compound 21 is cleaved with the particular enzyme and the liberated first fluorescent group 22 exhibits the fluorescence having the particular wavelength. Thus, when the fluorescence intensity in the sample solution is used as the basis, it can be detected to what extent the inhibitory substance for the deacetylase activity, which is the candidate compound for anticancer drugs is present in the sample solution, and the candidate substance of anticancer drugs, anti-tumor drugs and leukemia therapeutic drugs can be screened.

### (Embodiment 4)

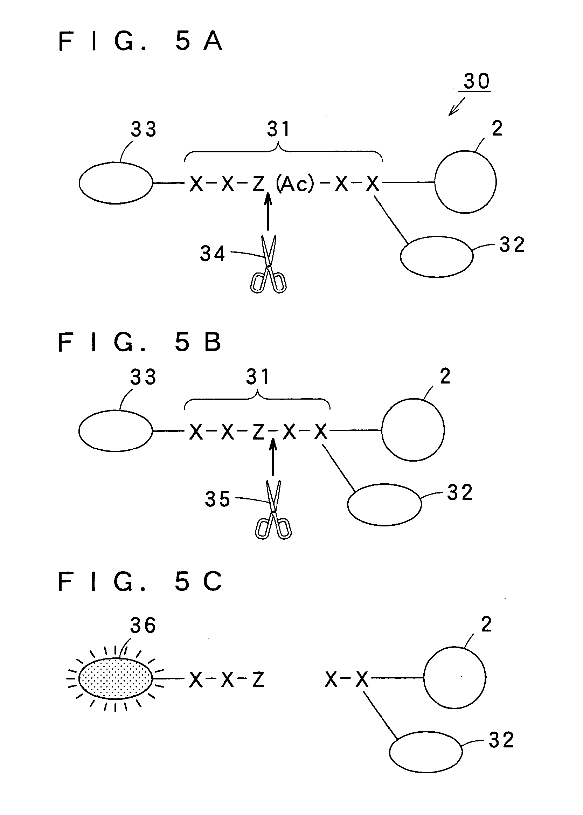

FIG. 5 is the schematic view showing the principle for detecting the enzyme activity of the particle for detecting the enzyme activity in Embodiment 4 of the present invention. The same symbols are given to the same items as in Embodiment 1, and description is omitted.
In the figure, the numeral 30 represents the particle for detecting the enzyme activity, and 31 represents the second compound such as an amino acid or a peptide bound to the surface of the particle 2 at one end. X in the second compound 31 represents the atomic group composed of any amino acid residue, and Z represents the acetylated amino acid residue such as acetylated lysine. The numeral 32 represents the quenching agent such as dinitrophenyl (Dnp) or 5-dimethylamino-1-naphthalene sulfonic acid (Dns) introduced into one end in the second compound 31, 33 represents the second fluorescent group such as (7-methoxycoumarin-4-yl)acetyl (MOAc) or tryptophan (Trp) introduced into the other end and bound to the quenching agent 32 at the distance (100 angstroms or less) in which they interact with each other to influence their fluorescence, 34 represents the deacetylase such as histone deacetylase in the sample solution, which catalyzes the reaction to liberate the acetyl group (Ac) from the acetylated amino acid residue Z, 35 represents the particular enzyme such as lysyl endopeptidase, plasmin, calpain, trypsin, metalloprotease or Armillaria mellea protease, which selectively cleaves the peptide bond in the C terminal side of the amino acid residue Z in the second compound 31, and 36 represents the second fluorescent group which is liberated by being cleaved with the particular enzyme 35 in the second compound 31 to change the fluorescence wavelength.

Concerning the particle for detecting the enzyme activity in Embodiment 4 constitutedas above, the principle for detecting the enzyme activity will be described below.
In the particle 30 for detecting the enzyme activity shown in FIG. 5A, the quenching agent 32 and the second fluorescent group 33 are bound at the distance in which they interact with each other to influence their fluorescence. Thus, the absorption spectrum of the quenching agent 32 and the fluorescence spectrum of the second fluorescent group 33 are overlapped. When the energy having the excitation wavelength of the second fluorescent group 33 is exposed, the fluorescence of the second fluorescent group 33 which should be inherently observed is attenuated.
When this particle 30 for detecting the enzyme activity is contacted and reacted with the sample solution containing the deacetylase 34 and the particular enzyme 35, the deacetylase 34 liberates the acetyl group (Ac) from the amino acid residue Z in the second compound 31. The particular enzyme 35 does not cleave the peptide bond or has a significantly lowered cleavage activity and exhibits almost no activity when the amino acid residue Z in the second compound 31 is acetylated.
When the acetyl group is liberated from the amino acid residue Z in the second compound 31, the particular enzyme 35 cleaves the peptide bond of the amino acid residue Z in the second compound 31 whose acetyl group (Ac) has been liberated as shown in FIG. 5B.
When the second fluorescent group 33 is liberated from the particle 2 into the sample solution, the interaction to influence the fluorescence between the second fluorescent group 33 and the quenching agent 32 is not observed. Thus, when the energy having the excitation wavelength of the second fluorescent group 33 is exposed to the sample solution, the fluorescence wavelength of the second fluorescent group 36, which has not been observed before contacting with the sample solution is observed, and is different from the fluorescence wavelength before the reaction with the sample solution. Therefore, the enzyme activity can be detected using the change in the fluorescence intensity as the indicator (see FIG. 5C) .
Accordingly, when the sample solution contains the inhibitory substance for the deacetylase activity, which inhibits the activity of the deacetylase 34, by measuring and comparing each fluorescence intensity in the case where the sample solution does not contain the inhibitory substance for the deacetylase activity, it is possible to determine whether the inhibitory substance for the deacetylase activity is effective.

The particle for detecting the enzyme activity in Embodiment 4 is constituted as above, and thus, the following action is obtained in addition to the actions described in Embodiment 3.
(1) In the second compound 31, the amino acid sequence can be freely selected and the particular enzyme 35 can also be selected freely. Thus, it is possible to use, as the particular enzyme 35, lysyl endopeptidase which is excellent in stability for selectively cleaving the peptide bond in the C terminal side, and enhance the enzyme detection accuracy.

### (Embodiment 5)

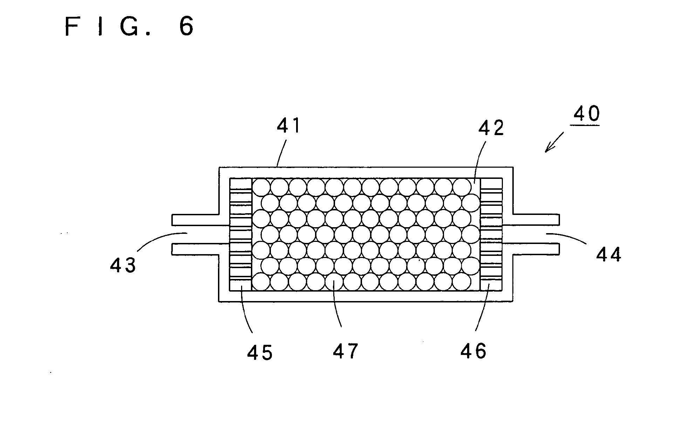

FIG. 6 is the schematic view showing the enzyme activity detection device in Embodiment 5 of the present invention.
In the figure, the numeral 40 represents the enzyme activity detection device, 41 represents the vessel formed into a cylindrical shape, 42 represents the liquidpassingpath formed in the vessel 41, 43 represents the liquid inlet formed upstream of the liquid passing path 42, 44 represents the liquid outlet formed downstream of the liquid passing path 42, 45 represents the filter formed into a porous shape and disposed at the liquid inlet 43, 46 represents the filter formed into the porous shape and disposed at the liquid outlet 44, and 47 represents the particle for detecting the enzyme activity, housed in the liquid passing path 42 inside the filters 45 and 46 and retained in the vessel 41. As the particle for detecting the enzyme activity, those described in Embodiments 1 to 5 can be used.

The method for using the enzyme activity detection device 40 constituted as above will be described below.
The sample solution such as urine is allowed to flow in the vessel 41 from the liquid inlet 43, pass through the liquid passing path 42 in the vessel 41 and discharge from the liquid outlet 44. The sample solution is contacted and reacted with the particles 47 for detecting the enzyme activity filled in the liquid passing path 42, and then discharged from the liquid outlet 44. Thus, the fluorescence intensity of the sample solution discharged from the liquid outlet 44 is measured.

Since the enzyme activity detection device in Embodiment 5 is constituted as above, the following actions are obtained.
(1) By measuring the fluorescence intensity in the sample solution discharged from the liquid outlet 44 after contacting and reacting with the particles 47 for detecting the enzyme activity, it is possible to simply detect whether the enzyme having the cleavage activity is contained in the sample solution. The sample solution can be measured without being influenced by the fluorescence from the particles 47 for detecting the enzyme activity. Thus, the present invention is excellent in flexibility because the fluorescent group can be selected without considering the fluorescence change caused by the liberation from the particles 47 for detecting the enzyme activity.
(2) Since the particle 47 for detecting the enzyme activity is filled and retained in the vessel 41, the new measurement can be performed by replacing the whole vessel 41 with a new one after finishing the measurement, and the handleability is excellent.

### Examples

The present invention will be described more specifically below by Examples, but the present invention is not limited to these Examples.
For the amino acids, peptides, protecting groups, solvents and the like described in the present Examples, abbreviations commonly used in the art or abbreviations employed by IUPAC-IUB Nomenclature are used. For example, the following abbreviations are used: Ala: alanine, Lys: lysine, β-Ala: β-alanine, Glu: glutamic acid, Gly: glycine, Arg: arginine, FITC: fluorescein-4-isothiocyanate isomer-1 (fluorescein isothiocyanate), DMF: N,N-dimethylformamide, DIEA: N,N-diisopropylethylamine, DCM: dichloromethane, i-PrOH: 2-propanol, MeOH: methanol, Lys (Dnp): (2S)-2-amino-6-(2,4-dinitrophenylamino)hexanoic acid, Pro: proline, Phe: phenylalanine, Leu: leucine, Val: valine, Asn: asparagine, Asp: aspartic acid, Tyr: tyrosine, Gln: glutamine, His: histidine, Ser: serine and Thr: threonine.

### (Example 1)

In Example 1, peptidyl fluorescent group-binding spherical particles were synthesized as the particles for detecting the enzyme activity, and the activity was measured by an enzyme, subtilisin. The method thereof will be described below.

### <Synthesis of peptidyl fluorescent group-binding spherical particles>

As the particle, commercially available spherical NH₂-PEGA resin (manufactured by Watanabe Chemical Industries Ltd., particle diameter: about 0.1 mm) was used. Five amino acids, Lys (Dnp), Zzz, Yyy, Xxx and β-Ala were introduced sequentially into the NH₂-PEGA resin (0.5g, 25µmol) using a peptide synthesizer (Model 433A, Applied Biosystems). Xxx, Yyy and Zzz are amino acids shown in Table 1. Subsequently, the particles were placed in a plastic vessel and swelled by adding DMF. After aspirating to remove DMF, FITC (30 µmol, 12 mg) dissolved in a small amount of DMF, DMF (2 mL) and DIEA (25 µmol, 4.4 µL) were added, and the mixture was shaken at room temperature for 3 hours. After aspirating to remove the reaction solution, the particles were washed with DMF (2 mL, twice), DCM (2 mL, twice), i-PrOH (2 mL, twice), DMF (2 mL, twice) , MeOH (2 mL, twice) and ether (2 mL, twice) in this order. Subsequently, the particles were reacted with a mixed solution of phenol (75 mg), 1,2-ethanedithiol (25 µL), thioanisole (50 µL), distilled water (50 µL) and TFA (2 mL) for 3 hours. After aspirating to remove the reaction solution, the particles were washed with DCM (2 mL, twice), DMF (2 mL, twice), 20% piperidine/DMF (2 mL, twice), DMF (2 mL, twice), i-PrOH (2 mL, twice), DMF (2 mL, twice), distilled water (2 mL, twice) and ether (2 mL, once) in this order, anddriedunder reducedpressure. These manipulations afforded eight types (samples 1 to 8 of amino acid sequences shown in Table 1) of particles for detecting the enzyme activity, having the second compound composed of β-Ala-Xxx-Yyy-Zzz-Lys where the second fluorescent group composed of FITC had been bound to β-alanine at one end and the quenching agent composed of dinitrophenyl (Dnp) had been bound to lysine at the other end, and the particle composed of PEGA resin bound to lysine in the second compound.

**[Table 1]**

| | Amino acid sequence | | | Fluorescence value |
|---|---|---|---|---|
| | Xxx | Yyy | Zzz | |
| Sample 1 | Pro | Leu | Gly | 14.8 |
| Sample 2 | Ala | Pro | Ala | 7.9 |
| Sample 3 | Ala | Pro | Val | 1.2 |
| Sample 4 | Ala | Pro | Leu | 13.9 |
| Sample 5 | Ala | Pro | Phe | 1.7 |
| Sample 6 | Asn | Leu | Asp | 13.2 |
| Sample 7 | Leu | Leu | Glu | 13.1 |
| Sample 8 | Leu | Val | Tyr | 1.1 |

### <Measurement of enzyme activity>

To each 1 mg of the particle for detecting the enzyme activity of the samples 1 to 8, 190 µL of 20 mM Tris-HCl buffer (pH 8.0, 100 mM NaCl, 50 mM CaCl₂) containing 0.01% Tween 20 was added, to which 10 µL of subtilisin aqueous solution (10 units) as a sample solution was added. Every measurement time, 10 µL of the sample was taken and 190 µL of the buffer was added, the mixture was transferred to a 96-well plate, and fluorescence values were measured (measurement time: 0.1 seconds) using Wallac 1420 ARVO sx plate reader manufactured by PerkinElmer. A changed amount (difference between the fluorescence value 20 minutes after adding the sample solution and the fluorescence value before adding the sample solution) of the fluorescence value 20 minutes after adding the sample solution is shown in the column on the right in Table 1 (excitation wavelength: 485 nm, fluorescence wavelength: 535 nm).
As shown in Table 1, 10 times or more difference in the changed amount of the fluorescence values is observed between sample 1 with highest activity and sample 8 with lowest activity. This has confirmed that it is possible to detect the difference in the subtilisin enzyme activity due to the each substrate using the particles for detecting the enzyme activity having a different amino acid sequence in the second compound.

### (Example 2)

In Example 2, peptidyl fluorescent group-binding spherical particles were synthesized as particles for detecting the enzyme activity, and the activity was measured by the enzyme, trypsin. The method thereof will be described below.

### <Synthesis of peptidyl fluorescent group-binding spherical particles>

Thirteen types (samples 9 to 21 of amino acid sequences shown in Table 2) of the particles for detecting the enzyme activity, having the second compound composed of β-Ala-Xxx-Yyy-Zzz-Lys where the second fluorescent group composed of FITC had been bound to β-alanine at one end and the quenching agent composed of dinitrophenyl (Dnp) had been bound to lysine at the other end, and the particles composed of PEGA resin bound to lysine in the second compound were synthesized in the same way as described in Example 1.

**[Table 2]**

| | Amino acid sequence | | | Fluorescence value |
|---|---|---|---|---|
| | Xxx | Yyy | Zzz | |
| Sample 9 | Gln | Arg | Glu | 197 |
| Sample 10 | Asp | Gly | Arg | 57 |
| Sample 11 | Pro | Arg | Gly | 51 |
| Sample 12 | His | Glu | Lys | 45 |
| Sample 13 | Ser | Ala | Arg | 24 |
| Sample 14 | Asn | Pro | Arg | 18 |
| Sample 15 | Gly | Lys | Arg | 18 |
| Sample 16 | Gly | Arg | Arg | 14 |
| Sample 17 | Pro | Arg | Leu | 10 |
| Sample 18 | Thr | Arg | Val | 9 |
| Sample 19 | Phe | Phe | Arg | 8 |
| Sample 20 | His | Leu | Lys | 7 |
| Sample 21 | Pro | Phe | Arg | 3 |

### <Measurement of enzyme activity>

To each 1 mg of the particle for detecting the enzyme activity of the samples 9 to 21, 190 µL of 20 mM Tris-HCl buffer (pH 8.0, 100 mM NaCl, 50 mM CaCl₂) containing 0.01% Tween 20 was added, to which 16 µL of trypsin aqueous solution (100 units) as the sample solution was added. Every measurement time, 10 µL of the sample was taken and 190 µL of the buffer was added, the mixture was transferred to the 96-well plate, and fluorescence values were measured (measurement time: 0.1 seconds) using Wallac 1420 ARVO sx plate reader manufactured by PerkinElmer. The changed amount (difference between the fluorescence value 60 minutes after adding the sample solution and the fluorescence value before adding the sample solution) of the fluorescence value 60 minutes after adding the sample solution is shown in the column on the right in Table 2 (excitation wavelength: 485 nm, fluorescence wavelength: 535 nm).
As shown in Table 2, 60 times or more difference in the changed amount of the fluorescence values is observed between sample 9 with highest activity and sample 21 with lowest activity. This has confirmed that it is possible to detect the difference in the trypsin enzyme activity due to the substrate using the particles for detecting the enzyme activity having a different amino acid sequence in the second compound.

### (Example 3)

In Example 3, peptidyl fluorescent group-binding spherical particles were synthesized as the particles for detecting the enzyme activity, and the enzyme activity was measured and compared among the enzymes. The method thereof will be described below.

### <Synthesis of peptidyl fluorescent group-binding spherical particles>

Four types (samples 22 to 25 of amino acid sequences shown in Table 3) of the particles for detecting the enzyme activity, having the second compound composed of β-Ala-Xxx-Yyy-Zzz-Lys where the second fluorescent group composed of FITC had been bound to β-alanine at one end and the quenching agent composed of dinitrophenyl (Dnp) had been bound to lysine at the other end, and the particles composed of PEGA resin bound to lysine in the second compound were synthesized in the same way as described in Example 1.

**[Table 3]**

| | Amino acid sequence | | | Fluorescence value | | |
|---|---|---|---|---|---|---|
| | Xxx I | Yyy | Zzz | Elastase | Trypsin | Subtilisin |
| Sample 22 | Leu | Leu | Glu | 38 | 2 | 481 |
| Sample 23 | Ala | Pro | Ala | 582 | 13 | 291 |
| Sample 24 | Asn | Leu | Asp | 23 | 5 | 446 |
| Sample 25 | Ala | Pro | Leu | 134 | 11 | 428 |

### <Measurement of enzyme activity>

To each 1 mg of the particle for detecting the enzyme activity of the samples 22 to 25, 190 µL of 20 mM Tris-HCl buffer (pH 8.0, 100 mM NaCl, 50 mM CaCl₂) containing 0.01% Tween 20 was added, to which 10 µL of elastase aqueous solution (10 units), 10 µL of trypsin aqueous solution (10 units) and 10 µL of subtilisin aqueous solution (10 units) as the sample solution was added. Every measurement time, 10 µL of the sample was taken and 190 µL of the buffer was added, the mixture was transferred to the 96-well plate, and fluorescence values were measured (measurement time: 0.1 seconds) using Wallac 1420 ARVO sx plate reader manufactured by PerkinElmer. The changed amount (difference between the fluorescence value 60 minutes after adding the sample solution and the fluorescence value before adding the sample solution) of the fluorescence value 60 minutes after adding the sample solution is shown in the column on the right in Table 3 (excitation wavelength: 485 nm, fluorescence wavelength: 535 nm).
As shown in Table 3, the fluorescence change by trypsin activity is about 1/240 of that by subtilisin activity in sample 22. The fluorescence change by elastase activity is about 2 times that by subtilisin activity in sample 23. This has confirmed that the particle for detecting the enzyme activity having a different amino acid sequence exhibits the different fluorescence change due to the difference in the substrate specificity.
From this, it has been demonstrated that the enzyme activities in the sample solution containing multiple enzymes can be measured and analyzed completely in a short time and the measurement efficiency can be exponentially enhanced, by placing the particles for detecting the enzyme activity having a different amino acid sequence in wells of the 96-well microplate for fluorescence measurement, subsequently injecting the sample solution in each well and performing the image analysis across a broad range using the image sensor.
Also, multiple types of the particles for detecting the enzyme activity having a different amino acid sequence are synthesized, and multiple enzyme activity detection devices housing respective particles for detecting the enzyme activity having a different amino acid sequence in respective vessels are prepared. For example, urine from the subj ects as the sample solutions are passed through the multiple enzyme activity detection devices, and the fluorescence intensity of the passed sample solutions was measured and recorded. By periodically and continuously, e.g. , once a week or every day measuring as above and comparing with the previous records, it is possible to detect whether the amount or the type of the enzyme contained in the urine has been changed, and it is possible to diagnose whether the physical condition has been changed using the fluorescence intensity in the sample solution contacted with the particles for detecting the enzyme activityas the indicator.

It has been described that the particles for detecting the enzyme activity where the second fluorescent group composed of fluorescein isothiocyanate (FITC) had been bound to one end and the quenching agent composed of dinitrophenyl (Dnp) had been bound to lysine at the other end, and the particles composed of PEGA resin bound to lysine in the second compound were synthesized, and that the difference in the enzyme activities can be detected by measuring the fluorescence change in the sample solutions contacted therewith. It was confirmed that when (7-methoxycoumarin-4-yl)acetyl (MOAc) was used as the second fluorescent group, the same result was also obtained.
When using the particle for detecting the enzyme activity comprising the first compound such as substrate peptide binding the particle such as PEGA resin at one end, binding the first fluorescent group such as FITC at the other end and having the cleavage site by the enzyme, it was also confirmed that the difference in the enzyme activities can be detected by measuring the fluorescence change in the sample solutions contacted therewith.
The particle for detecting the enzyme activity where lysine (Yyy) in the second compound in sample 15 described in Example 2 hadbeen acetylatedwas synthesized, this was contacted and reacted with the sample solution containing the histone deacetylase and the particular enzyme such as metalloprotease, subsequently the fluorescence in the sample solutions was measured, and the measurement values were compared. As a result, it was confirmed that the fluorescence change was small in the sample solution abundantly containing the inhibitory substance for the histone deacetylase activity. From this, it has been demonstrated that by using the particle for detecting the enzyme activity of the Examples, it is possible to detect to what extent the inhibitory substance for the histone deacetylase activity, which is the candidate compound for the anticancer drug, etc. is present in the sample solution and easily and promptly screen the candidate substance for the anticancer drug, etc..

### Industrial Applicability

The present invention relates to a particle for detecting an enzyme activity, which detects the enzyme activity, and the method for detecting the enzyme activity and an enzyme activity detection device by the use thereof. Bymeasuring the intensity of the fluorescence wavelength in the sample solution, it is possible to not only detect the presence or absence of the enzyme but also perform quantitative analysis of the enzyme in the sample solution with high accuracy. The present invention is hardly influenced by absorbed moisture and weighing errors hardly occur, and thus, is excellent in handleability as well as enhancing the measurement accuracy and the quantitative property. The present invention can provide the particle for detecting enzyme activity which is remarkably excellent in productivitybecause complicated steps suchas a step of cleaving and purifying the synthesized peptide and a step of lyophilizing are not required. The enzyme activity can be detected only by measuring the fluorescence intensity in the sample solution after contacting the particle with the sample solution containing the enzyme. The present invention can shorten the measurement time, enhances the workability and is excellent in measurement efficiency. By performing the image analysis across a broad range using the image sensor, it is possible to completely measure and analyze the enzyme activities in the sample solution containing multiple enzymes in a short time and enhance the measurement efficiency exponentially. Thus, the present invention can provide the method for detecting the enzyme activity, in which useful substances leading to pharmaceuticals can be searched and screened highly efficiently. It is also possible to provide the enzyme activity detection device which can easily detect whether the enzyme having the activity is contained in the sample solution by measuring the fluorescence intensity in the sample solution, and is excellent in handleability.

## Claims

1. A particle for detecting an enzyme activity comprising a particle and a first compound binding the particle to one end, binding a first fluorescent group to the other end and having a cleavage site by an enzyme.

2. The particle for detecting the enzyme activity according to claim 1, wherein the first fluorescent group is fluorescein or a derivative thereof.

3. A particle for detecting an enzyme activity comprising a second compound binding a second fluorescent group to one end, binding a quenching agent to an atomic group at the other end and having a cleavage site by an enzyme, and a particle bound to the atomic group or the quenching agent at the other end in the second compound.

4. The particle for detecting the enzyme activity according to any one of claims 1 to 3, wherein the first compound or the second compound has an acetylated amino acid residue.

5. A method for detecting an enzyme activity comprising (a) a contact reaction step of contacting and reacting a sample solution containing an enzyme with the particle for detecting the enzyme activity according to any one of claims 1 to 3, and (b) a fluorescence measurement step of measuring fluorescence in the sample solution reacted with the particle for detecting the enzyme activity.

6. A method for detecting an enzyme activity comprising (a) a contact reaction step of contacting and reacting the particle for detecting the enzyme activity of claim 4 with a sample solution containing a deacetylase which liberates an acetyl group from the first compound or the second compound having an acetylated amino acid residue and a particular enzyme which selectively cleaves a peptide bond of the amino acid residue whose acetyl group has been liberated, and (b) a fluorescence measurement step of measuring fluorescence in the sample solution reacted with the particle for detecting the enzyme activity.

7. An enzyme activity detection device comprising a vessel comprising a liquid inlet formed upstream of a liquid passing path and a liquid outlet formed downstream of the liquid inlet and the particle for detecting the enzyme activity according to any one of claims 1 to 4 housed in the liquid passing path and retained in the vessel.
